# DEMANDE DE BREVET EUROPEEN

(11) **EP 0 862 923 A1**
(43) Date de publication de la demande: **09.09.1998**
(21) Numéro de dépôt: 98410022.2
(22) Date de dépôt: 02.03.1998
(51) Int. Cl.: A61M 16/00, A61M 16/04

(54) **Dispositif d'intubation pour la respiration artificielle d'un patient**

(30) Priorité: 05.03.1997 FR 9702821
(71) Demandeur: R.A.O. Modelage-Maquettes, 73310 Motz (FR)
(72) Inventeur: Carasco, Bernard André, 73310 Motz (FR)
(74) Mandataire: Hecké, Gérard

(57) **Abrégé**

L'invention est relative à un dispositif d'intubation utilisé pour la respiration artificielle en réanimation ou en anesthésie générale, et comprenant une canule 12 dotée d'un tube de maintien 16 incurvé et creux en matière plastique semi-rigide, introduit dans la bouche au-dessus de la langue jusqu'au fond de la gorge du patient, et un tube d'intubation 14 inséré dans la trachée du patient pour assurer la respiration artificielle. La canule 12 est associée à une embase de fixation 22 équipée d'un trou 26 central en communication avec le canal interne du tube de maintien 16, et de moyens d'encliquetage 28, 30 du tube d'intubation 14 sur au moins un côté de ladite embase.

## Description

L'invention est relative à un dispositif d'intubation utilisé pour la respiration artificielle en réanimation ou en anesthésie générale, et comprenant:
- une canule dotée d'un tube de maintien incurvé et creux en matière plastique semi-rigide, introduit dans la bouche au-dessus de la langue jusqu'au fond de la gorge du patient,
- un tube d'intubation inséré dans la trachée du patient pour assurer ladite respiration artificielle,
- une embase de fixation solidaire de la canule et équipée d'un trou central en communication avec le canal interne du tube de maintien, et de moyens d'encliquetage du tube d'intubation sur au moins un côté de ladite embase.

L'usage des canules en anesthésie permet de maintenir un passage constant dans la gorge, pour d'une part éviter un éventuel retournement de la langue lors de la période d'anesthésie, et d'autre part autoriser la mise en place du tube d'intubation dans la trachée du patient. Il existe des canules de différentes tailles en fonction de l'âge et de la morphologie du patient, chaque canule coopérant de plus avec un bouchon tubulaire réalisé en un matériau plastique, lequel est inséré dans le tube de maintien de la canule pour rigidifier la zone sur laquelle portent les dents. La présence de ce bouchon évite tout écrasement du tube de maintien en cas de serrage des mâchoires du patient. Dans les dispositifs connus, le tube d'intubation est séparé de la canule à l'intérieur de la bouche, l'ensemble étant maintenu en place depuis l'extérieur au moyen de bandes adhésives et/ou d'un lien d'attache. La mise en oeuvre par le praticien d'un tel système d'intubation reste problématique en fonction des différentes morphologies des patients, et ne tient pas compte du confort durant l'intervention. L'usage de bandes adhésives non aseptisées pour fixer le tube d'intubation peut également poser des problèmes d'allergie et d'infection.

Le document US 5253643 décrit un dispositif d'intubation, dans lequel l'embase de fixation fait partie intégrante de la canule.

L'objet de l'invention consiste à réaliser un dispositif d'intubation sûr et efficace pouvant être mis facilement en place par le praticien, indépendamment de la morphologie du patient.

Le dispositif d'intubation selon l'invention est caractérisé en ce que la canule et l'embase de fixation sont formées par deux pièces distinctes assemblées l'une à l'autre par des moyens de liaison.

La canule du dispositif d'intubation peut se présenter sous la forme d'une ou de plusieurs pièces.

Selon un mode de réalisation préférentiel, la canule et l'embase de fixation sont formées par deux pièces distinctes assemblées l'une à l'autre par un bouchon creux, destiné à s'emmancher dans le tube de maintien, ledit bouchon étant réalisé en un matériau plastique ayant une rigidité mécanique supérieure à celle du tube de maintien de la canule.

Le bouchon peut être solidarisé à l'embase de fixation en faisant saillie vers le bas pour s'introduire dans un orifice d'une pièce de butée de la canule. Le bouchon peut également être formé par une pièce distincte, susceptible d'être introduite dans le trou de l'embase de fixation, et dans l'orifice de la pièce de butée de la canule.

D'autres avantages et caractéristiques ressortiront plus clairement de la description qui va suivre d'un mode de réalisation de l'invention, donné à titre d'exemple non limitatif, et représenté aux dessins annexés dans lesquels:
- la figure 1 est une vue éclatée en perspective du dispositif d'intubation selon l'invention;
- les figures 2 à 5 montrent diverses formes de réalisation de la canule équipant le dispositif d'intubation;
- la figure 6 représente le dispositif d'intubation de la figure 1 en position montée, avec l'encliquetage du tube d'intubation sur le côté droit de l'embase de fixation;
- la figure 7 est une vue identique de la figure 6 avec le montage du tube d'intubation sur le côté gauche;
- la figure 8 montre le dispositif d'intubation mis en place dans la bouche d'un patient;
- les figures 9 et 10 représentent deux autres variantes de réalisation de la canule.
- la figure 11 est une vue identique de la figure 6 d'une autre variante du dispositif d'intubation sans canule.

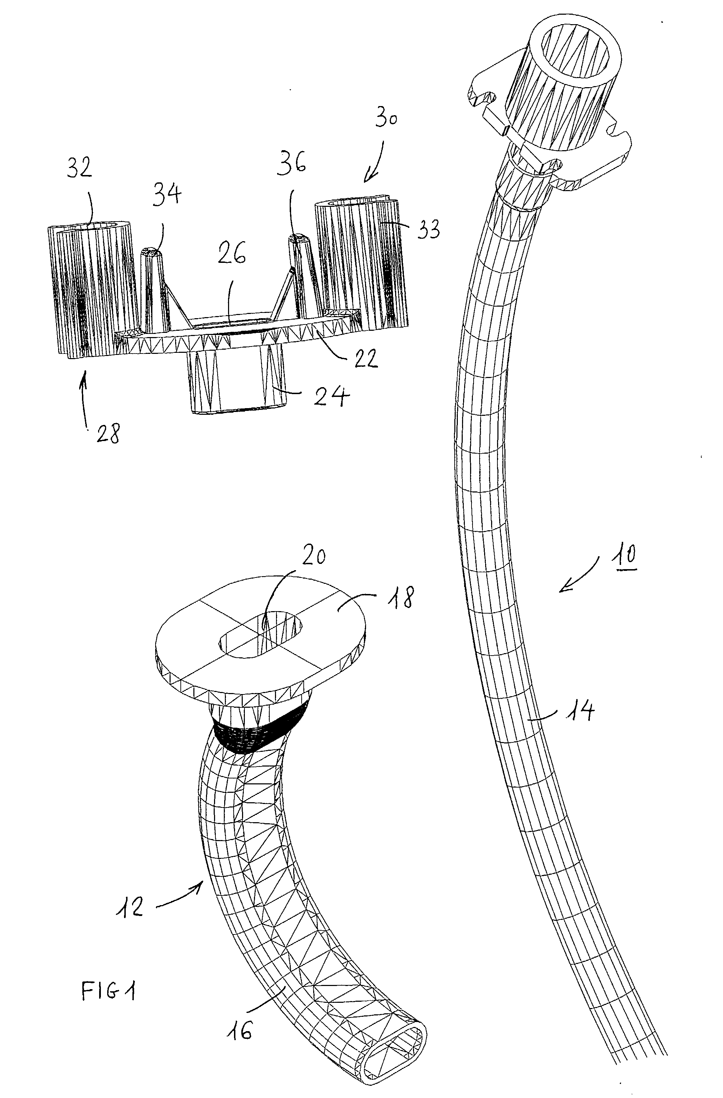

En référence à la figure 1, un dispositif d'intubation 10 utilisé pour la respiration artificielle en réanimation ou en anesthésie générale, comporte une canule 12 associée à un tube d'intubation 14. La canule 12 est formée par un tube de maintien 16 creux en matière plastique semi-rigide, et présentant une forme incurvée destinée à être introduite dans la bouche au-dessus de la langue, et jusqu'au fond de la gorge du patient, en regard des voies respiratoires. La partie antérieure du tube 16 est élargie par une pièce de butée 18 en forme de plaque venant se placer sur l'extérieur des lèvres lorsque la canule 12 est mise en place dans la bouche. La pièce de butée 18 comporte un orifice 20 central en communication avec l'intérieur du tube 16.

Une embase de fixation 22 peut être adaptée à la pièce de butée 18 de la canule 12 par emmanchement d'un bouchon 24 creux dans l'orifice 20. Le bouchon 18 sert de moyen d'assemblage de l'embase 22 à la canule 12, et simultanément de moyen de rigidification du tube 16 dans la zone frontale sur laquelle portent les dents du patient.

L'embase 22 en matériau plastique est pourvue d'un trou 26 agencé au droit du bouchon 24 creux, de manière à venir en regard de l'orifice 20 lors de l'assemblage de l'embase 22 avec la canule 12. Des moyens d'encliquetage 28, 30 équipent les extrémités latérales opposées de l'embase de fixation 22 pour permettre l'enclipsage du tube d'intubation 14 sur le côté gauche ou droit. Le bouchon 24 est réalisé en un matériau plastique rigide, ayant une rigidité mécanique nettement supérieure à celle du tube 16 de la canule 12.

Les moyens d'encliquetage 28, 30 sont symétriques par rapport au plan médian passant par le trou 26 de l'embase 22, et présentent chacun un embout de clipsage 32, 33 en forme de U et à entrée déformable, dirigée vers l'extérieur et à l'opposé du trou 26. Les deux embouts de clipsage 32, 33 viennent directement de moulage avec l'embase 22, et permettent le clipsage du tube d'intubation 14 contre la canule 12.

L'embase 22 comprend de plus deux ergots d'accrochage 34, 36 s'étendant entre les embouts de clipsage 32, 33, et le trou 26, et autorisant le passage d'une sangle d'attache (non représentée) destinée à entourer la tête du patient pour maintenir le dispositif d'intubation 10 en position stable. L'ensemble des pièces constitutives de la canule 12 et de l'embase de fixation 22 peut être réalisé selon différentes manières, en une ou plusieurs pièces.

Sur la figure 2, le bouchon 24 creux est solidaire de l'embase de fixation 22, et forme une seule pièce plastique moulée avec cette dernière, et les deux embouts de clipsage 32, 33. La canule 12 est identique à celle de la figure 1.

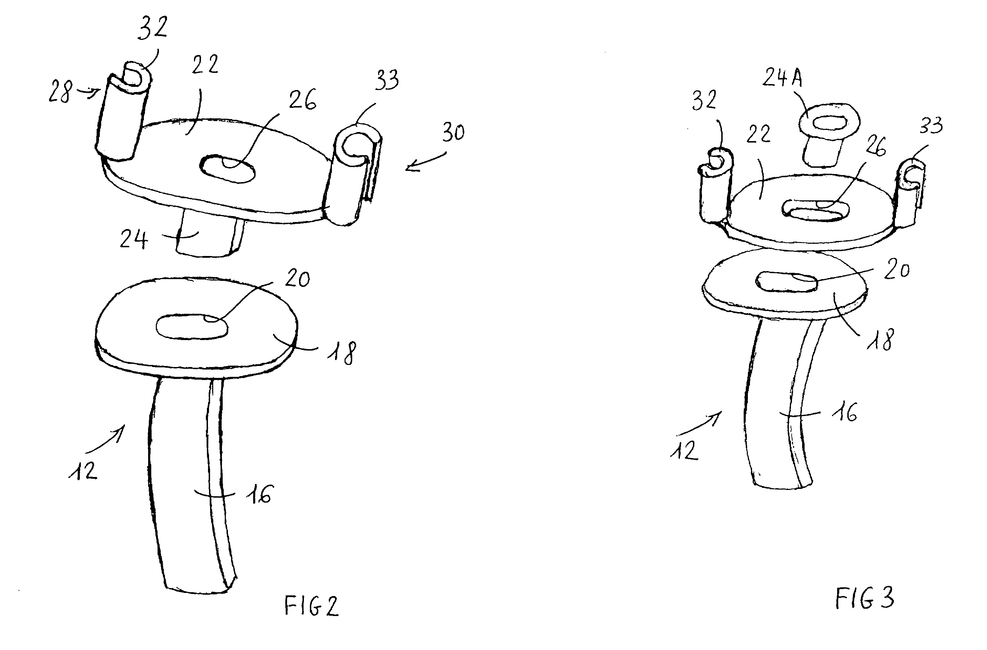

Sur la figure 3, le bouchon 24A creux est formé par une pièce distincte de l'embase de fixation 22, et enfichable à travers le trou 26, et l'orifice 20 pour assurer l'assemblage de l'embase 22 à la pièce de butée 18 de la canule 12. Le bouchon 24A comporte à cet effet un rebord 38 ayant un diamètre supérieur à celui du trou 26, de manière à prendre appui sur la face supérieure de l'embase 22. Une matière plastique spécifique peut être utilisée pour l'injection plastique de chaque pièce 24A, 22, et 12.

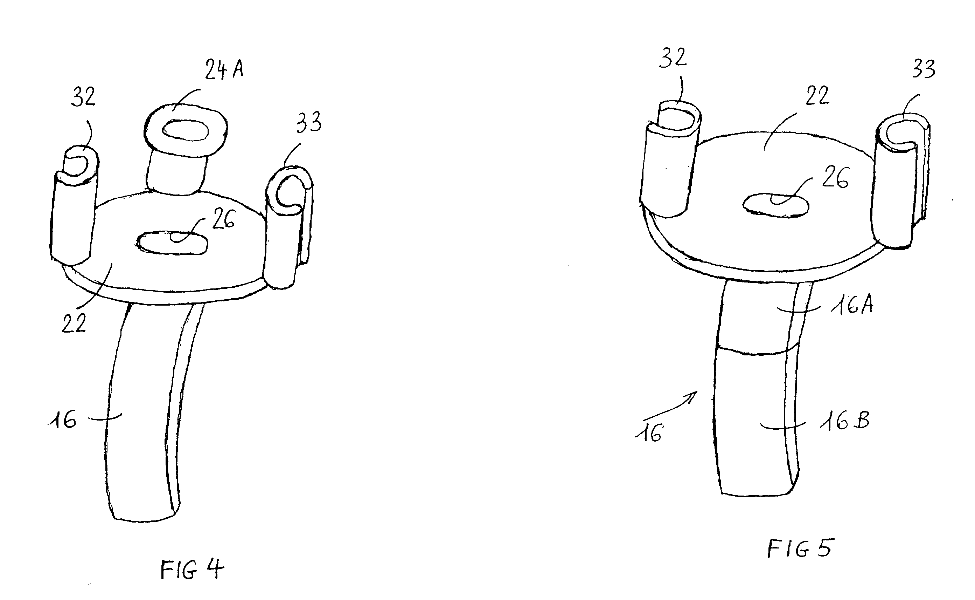

En référence à la figure 4, la pièce de butée 18 de la canule 12 est supprimée, et le tube de maintien 16 semi-rigide est assujeti directement à l'embase de fixation 22 équipée des deux embouts de clipsage 32, 33. Le bouchon 24A creux est introduit dans le tube 16 à travers le trou 26 d'une manière identique à celle de la figure 3.

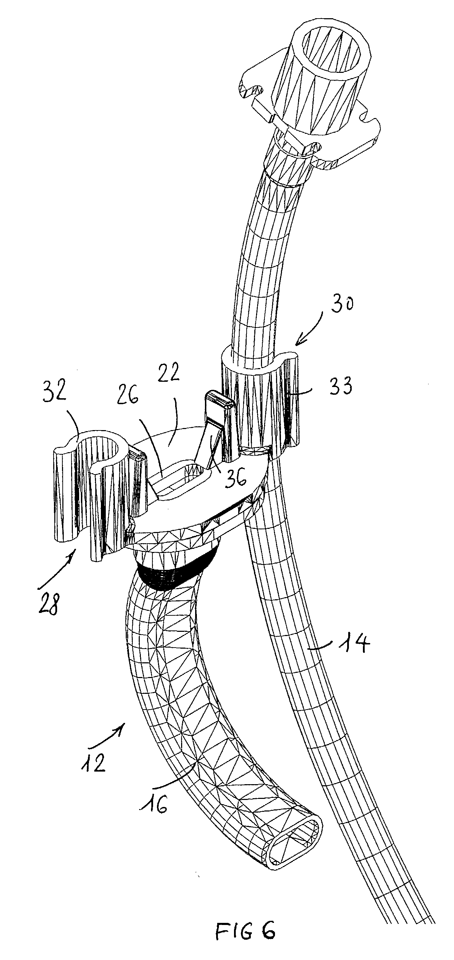

Sur la figure 5, une seule pièce remplit la fonction de canule et d'embase de fixation. Le bouchon 24A de la figure 4 est supprimé, et le tube 16 de la canule est subdivisé en une première partie 16A rigide, et une deuxième partie 16B souple ou semi-souple. Cette pièce monobloc est avantageusement obtenue par un moulage bi-matière, le matériau de la première partie 16A étant identique à celui de l'embase de fixation 22.

Sur la figure 6, le dispositif d'intubation 10 de la figure 1 est en position montée, et le tube d'intubation 14 est attaché à l'embout de clipsage 33 de droite.

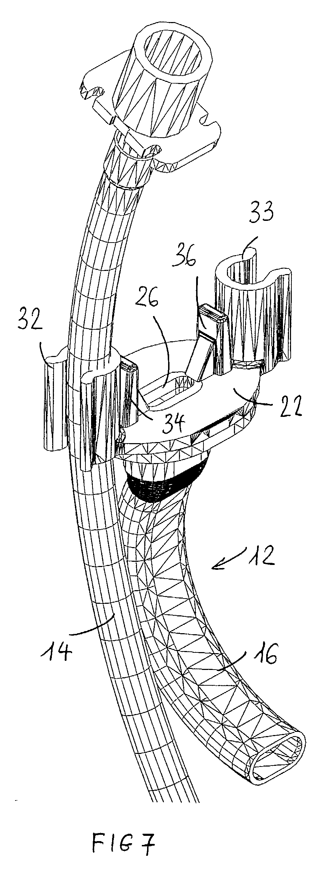

Sur la figure 7, le tube d'intubation 14 est fixé à l'embout de clipsage 32 de gauche.

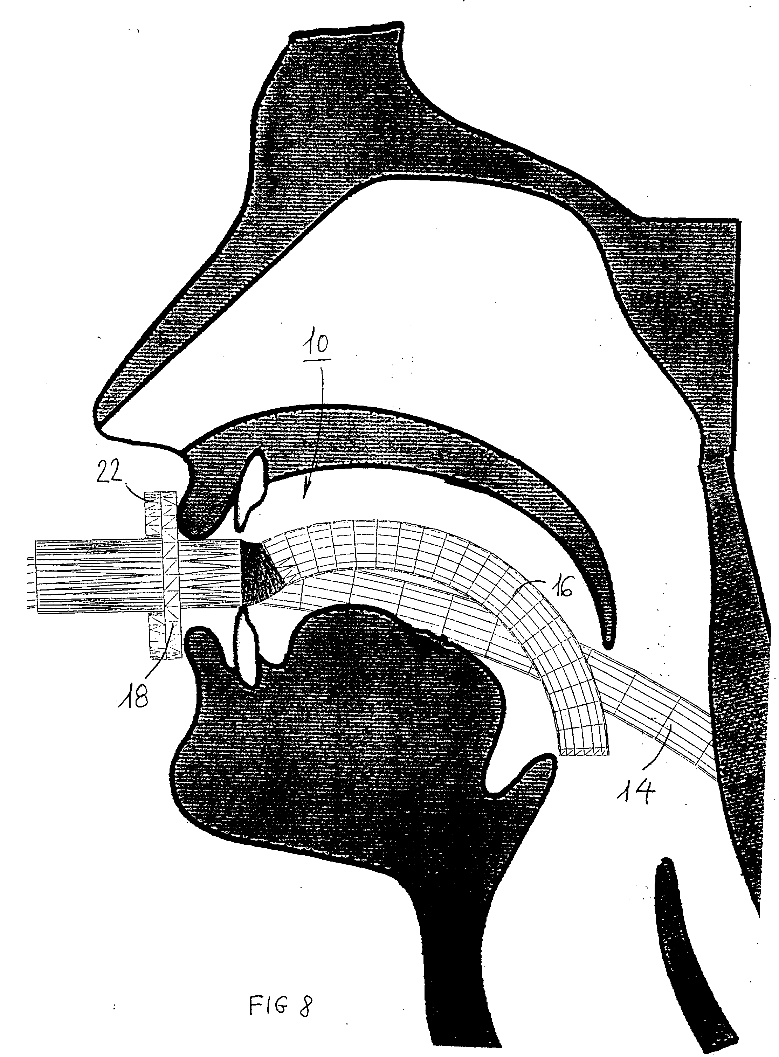

Sur la figure 8, le dispositif d'intubation 10 selon l'une des figures 6 et 7, est mis en place dans la bouche, avec introduction du tube 14 dans la trachée du patient pour autoriser la respiration artificielle pendant l'anesthésie.

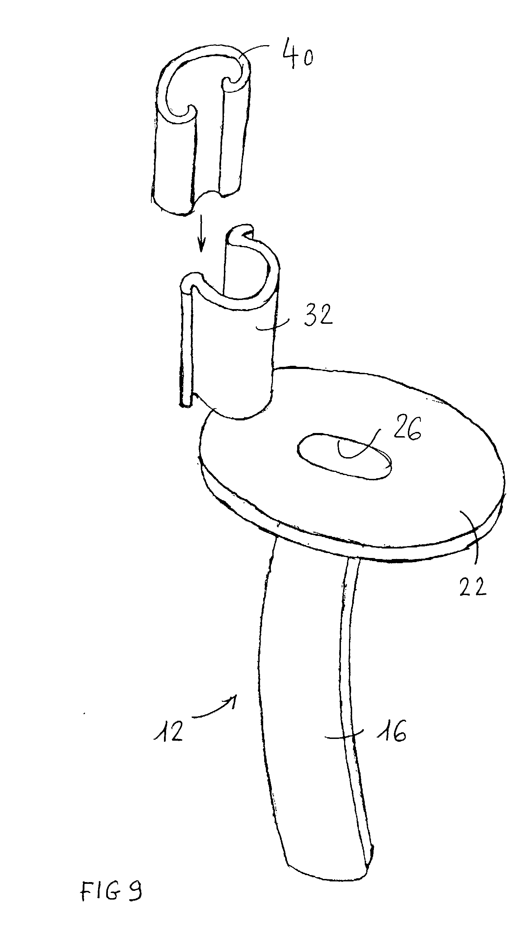

En référence à la figure 9, l'embout de clipsage 32 en forme d'oméga à rebords d'encliquetage, peut être obturé après mise en place du tube d'intubation (non représenté) au moyen d'un élément de verrouillage 40 destiné à s'emboîter sur les rebords d'encliquetage de l'embout 32. La présence de l'élément de verrouillage 40 évite tout échappement du tube d'intubation après son introduction dans la trachée.

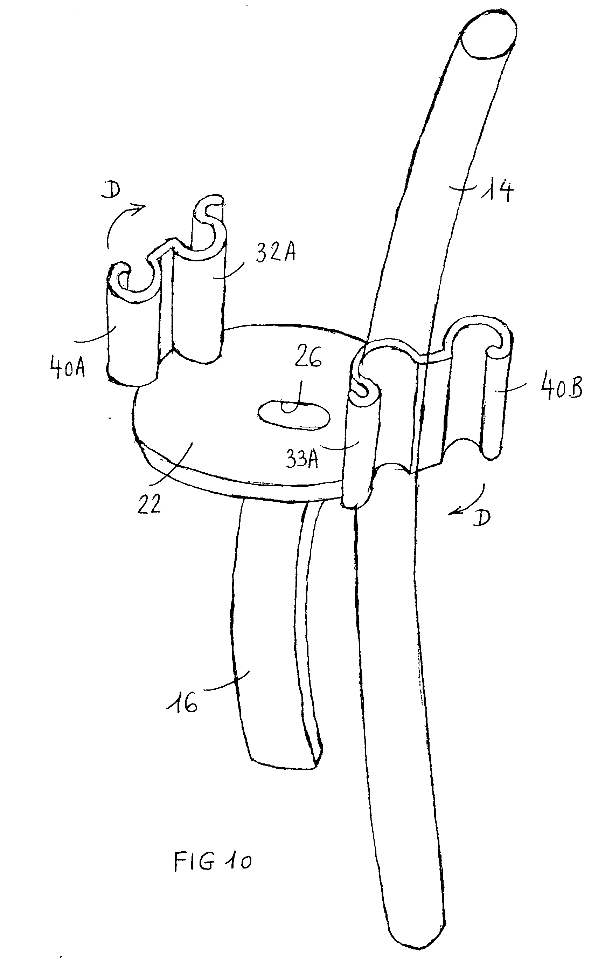

Selon la variante de la figure 10, chaque embout de clipsage 32A, 33A et l'élément de verrouillage 40A, 40B correspondant sont formés par une seule pièce obtenue par moulage plastique. Une ligne de résistance mécanique réduite est agencée dans la zone médiane pour constituer une charnière, autorisant le pivotement de l'élément de verrouillage 40A, 40B dans le sens de la flèche D pour assurer l'enclipsage sur l'embout de clipsage 32A, 33A correspondant.

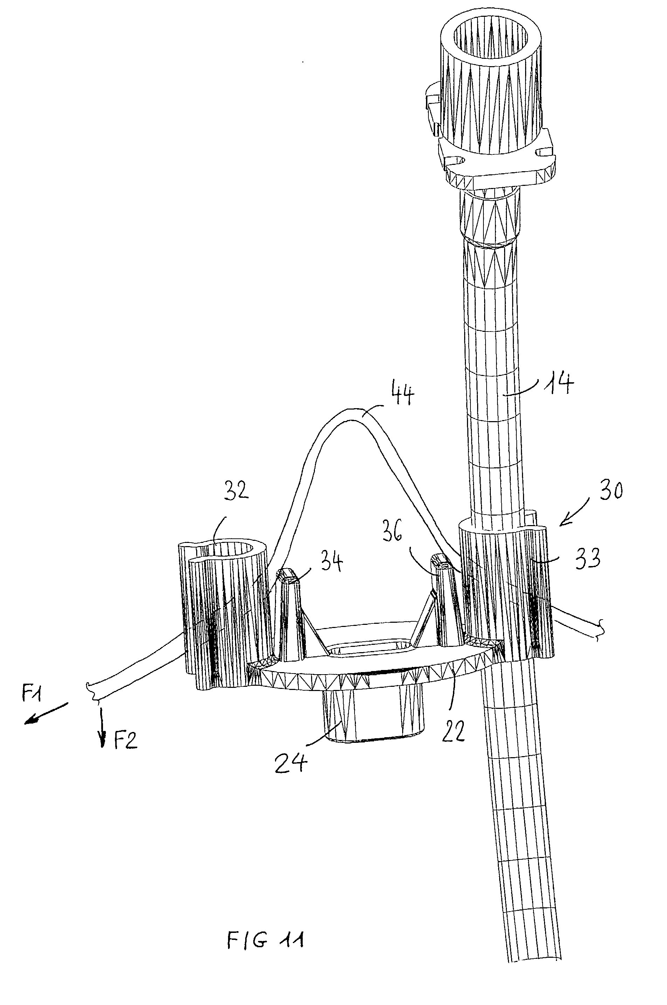

Sur la figure 11, une sangle d'attache 44 peut être ajustée en longueur et bloquée sur les ergots d'accrochage 34, 36 de l'embase de fixation 22 pour adapter le dispositif d'intubation autour de la tête du patient. Le réglage en longueur s'effectue en tirant sur l'extrémité de la sangle 44 dans le sens de la flèche F1, tandis que le blocage intervient en la tirant vers le bas dans le sens de la flèche F2 pour coincer la sangle 44 contre chaque ergot d'accrochage 34, 36 de forme conique. Le bouchon 24 associé à l'embase de fixation 22 est utilisé sans la canule 12.

## Revendications

1. Dispositif d'intubation utilisé pour la respiration artificielle en réanimation ou en anesthésie générale, et comprenant:
- une canule (12) dotée d'un tube de maintien (16) incurvé et creux en matière plastique semi-rigide, introduit dans la bouche au-dessus de la langue jusqu'au fond de la gorge du patient;
- un tube d'intubation (14) inséré dans la trachée du patient pour assurer ladite respiration artificielle,
- une embase de fixation (22) solidaire de la canule (12) et équipée d'un trou (26) central en communication avec le canal interne du tube de maintien (16), et de moyens d'encliquetage (28, 30) du tube d'intubation (14) sur au moins un côté de ladite embase,
caractérisé en ce que la canule (12) et l'embase de fixation (22) sont formées par deux pièces distinctes assemblées l'une à l'autre par des moyens de liaison.

2. Dispositif d'intubation selon la revendication 1, caractérisé en ce que les moyens de liaison comportent un bouchon (24, 24A) creux destiné à s'emmancher dans le tube de maintien (16), ledit bouchon étant réalisé en un matériau plastique ayant une rigidité mécanique supérieure à celle du tube de maintien (16) de la canule (12).

3. Dispositif d'intubation selon la revendication 2, caractérisé en ce que le bouchon (24) est solidarisé à l'embase de fixation (22) en faisant saillie vers le bas pour s'introduire dans un orifice (20) d'une pièce de butée (18) de la canule (12).

4. Dispositif d'intubation selon la revendication 2, caractérisé en ce que le bouchon (24A) est formé par une pièce distincte susceptible d'être introduite dans le trou (26) de l'embase de fixation (22), et dans l'orifice (20) d'une pièce de butée (18) de la canule (12).

5. Dispositif d'intubation selon la revendication 1, caractérisé en ce que le tube de maintien (16) de la canule (12) comporte une première partie (16A) rigide, et une deuxième partie (16B) souple ou semi-souple, l'ensemble formant une pièce plastique monobloc obtenue par moulage bi-matière.

6. Dispositif d'intubation selon la revendication 1, caractérisé en ce que les moyens d'encliquetage (28, 30) comportent au moins un embout de clipsage (32, 33, 32A, 33A) agencé à la périphérie de l'embase (22), et présentant une entrée déformable agencée à l'opposé du trou (26).

7. Dispositif d'intubation selon la revendication 6, caractérisé en ce que l'embase de fixation (22) comporte deux embouts de clipsage disposés symétriquement par rapport au plan médian passant par le trou (26) pour autoriser l'emboîtement du tube d'intubation (14) à gauche ou à droite.

8. Dispositif d'intubation selon la revendication 6 ou 7, caractérisé en ce que l'embout de clipsage (32, 33, 32A, 33A) coopère avec un élément de verrouillage (40, 40A, 40B) pour assurer le blocage du tube d'intubation (14) sur l'embase de fixation (22).
